# EUROPEAN PATENT APPLICATION

(11) **EP 0 795 610 A1**
(43) Date of publication of application: **17.09.1997**
(21) Application number: 96103951.8
(22) Date of filing: 13.03.1996
(51) Int. Cl.: C12Q 1/68, C12Q 1/70, G01N 33/50

(54) **In situ hybridization signal amplification based on biotinylated tyramine deposition**

(71) Applicant: Becton Dickinson and Company, Franklin Lakes, New Jersey 07417-1880 (US)
(72) Inventor: Kerstens, H.M.J., Department of Pathology, 6525 GA Nijmegen (NL); Poddighe, P.J., Department of Pathology, 6525 GA Nijmegen (NL); Hanselaar, A.G.J.M., Department of Pathology, 6525 GA Nijmegen (NL)
(74) Representative: Frohwitter, Bernhard, Dipl.-Ing.

(57) **Abstract**

According to the present invention, a method is provided for the detection and cellular localization of DNA or RNA sequences. In one embodiment, the method comprises the steps of labeling a DNA probe with a hapten, for example biotin, immunochemically detecting the hybridized DNA probe using, for example, a horseradish peroxidase (HRP)-labeled antibody, depositing labeled tyramine, e.g. by enzymatic action of HRP, thereby creating an additional biotin source, and detecting the additional biotin source with avidin biotin complex (ABC), enzyme, or flouorochrome avidin. In a further embodiment, a method is provided for detecting human papilloma virus by in situ hybridization signal amplification based on labeled tyramine deposition.

## Description

### Field of the Invention

The invention generally relates to in situ hybridization ("ISH") techniques for the detection and cellular localization of DNA or RNA sequences. More particularly, the invention is directed to methods for amplifying ISH signals by labeled tyramine deposition. Still more particularly, the invention is related to methods for detecting human papilloma virus using amplification based on labeled tyramine deposition.

### Related Art

Techniques for detecting nucleic acid sequences are becoming increasingly important as medical technology advances. For example, many viral infections may be treated, or cured, if discovered early enough. Moreover, some viral infections have been linked with the development of other afflictions which may also be treated if they are timely detected. For example, infection with certain human papilloma virus (HPV) types, especially types 16 and 18, has been postulated to be a high-risk factor for the development of human squamous cell carcinoma of the uterine cervix. See, e.g., Koss, L.G. Carcinogenesis in the Uterine Cervix and Human Papillomavirus Infection, In: K. Syrjänen, L. Gissman and L.G. Koss (eds.), Papillomaviruses and Human Diseases, Heidelberg, FRG: Springer Verlag (1988). Carcinoma of the cervix uteri is one of the most common types of cancer in the developing world. However, this type of cancer can be cured if detected in an early phase of the disease. Therefore, it is important that fast accurate techniques be devised to detect the presence of nucleic acid sequences.

One technique is known as polymerase chain reaction ("PCR"). With PCR it is possible to rapidly detect a viral infection, such as HPV, if only a few cells in a given tissue or cytological sample contain the DNA sequence of interest. One of the major disadvantages of PCR analysis is that no direct correlation with morphology can be performed because the DNA must be extracted from the tissue or cells of interest, and thus the architecture of the cells or tissue is lost. Therefore, the application of DNA-ISH techniques for the detection of HPV sequences is preferred to enable the correlation between the presence of HPV-DNA and the morphological aspects of the cells or tissues.

A PCR-ISH technique has been developed that combines the extreme sensitivity of PCR amplification and ISH. This technique has been applied to cell lines and paraffin-embedded, formalin-fixed tissues for the detection of HPV DNA. *See*, Nuovo, *et al*., An Improved Technique for the In Situ Detection of DNA after Polymerase Chain Reaction Amplification, Am. J. Pathol. 139: 1239-1244 (1991a), and Nuovo, G.J., *et al*., Detection of Human Papillomavirus DNA in Formalin-Fixed Tissues by In Situ Hybridization after Amplification by Polymerase Chain Reaction, Am. J. Pathol. 139: 847-854 (1991b). However, disadvantages of this procedure are: (1) it can lead to false positive results due to aspecific PCR reactions, (2) results are difficult to analyze, and (3) it is laborious and not routinely applicable.

To overcome the problems with PCR, other in situ hybridization techniques have been used as important tools to detect DNA target sequences. With these techniques, even very small DNA targets of 1-5 kb can be localized in metaphase spreads. Several techniques are used to visualize the DNA targets. One technique is known as fluorescence in situ hybridization ("FISH"). This technique, however, requires highly sensitive charge couple device ("CCD") cameras and sophisticated image enhancement equipment.

Another technique, known as brightfield in situ hybridization ("BRISH"), uses horseradish peroxidase ("HRP") labeled avidin-biotin complex ("ABC"). This technique is routinely applied for demonstration of DNA targets in formalin-fixed, paraffin-embedded tissues. However, only relatively large DNA targets with high repetitive sequences can be visualized by BRISH. For visualization of smaller DNA targets, the sensitivity of the ABC technique is too low, resulting in little or no detectable ISH signals.

Therefore, efforts have been made to amplify both FISH and BRISH techniques. For example, one method uses modification of the immunochemical detection of the probe label, such as silver intensification of diaminobenzidine ("DAB") precipitate, or amplification of fluorescent signals. However, common amplification methods are not completely satisfactory because they suffer from disadvantages such as increases in background staining, less target localization, and loss of morphology.

### SUMMARY OF THE INVENTION

According to the present invention, a method is provided for the detection and cellular localization of nucleic acids, i.e., DNA or RNA sequences. For purposes of illustration, the embodiments of the invention will be described primarily with respect to DNA. It is to be understood that in alternative embodiments, the invention detects RNA sequences as well. In one embodiment, the method comprises the steps of (1) labeling a DNA probe with a hapten, e.g., biotin, digoxygenin, or fluorescein; (2) hybridizing the DNA probe with target DNA; (3) applying an enzyme-labeled detection system, e.g., horseradish peroxidase ("HRP")-labeled avidin-biotin complex ("ABC") system; (4) depositing labeled tyramine through enzymatic action, thereby creating an additional label source; and, if necessary, (5) detecting the additional label source with fluorochrome or enzyme-labeled avidin.

In a further specific embodiment, a method for detecting human papilloma virus ( 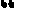 HPV 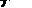 ) by in situ hybridization signal amplification is provided which comprises the steps of: (1) labeling DNA probes specific for a type of HPV to be detected, the labeling comprising e.g. nick-translation with label; (2) hybridizing the DNA probes with a target DNA; (3) immunochemically detecting the hybridized DNA probes with an enzyme-labeled detection system such as HRP-labeled avidin-label complex ( 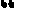 ABC 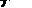 ); (4) applying labeled tyramine to deposit additional label source; (5) detecting the additional label source, for example, by reapplying ABC; and (6) visualizing the hybridized DNA.

### BRIEF DESCRIPTION OF THE FIGURES

Figure 1 is a schematic representation of a CARD-ISH technique according to an embodiment of the invention.

Figures 2A-D illustrate the results of CARD amplification brightfield ISH on tissue sections.

Figures 3A-G illustrate the results of CARD amplification in fluorescence ISH on metaphase spreads, according to an embodiment of the invention.

Figures 4A-D illustrate the results of CARD amplification in brightfield ISH on metaphase spreads, according to still a further embodiment of the invention.

Figure 5 shows the results of CARD-ISH signal amplification on cervical cancer cell lines according to one embodiment of the invention.

Figure 6 shows the results of CARD-ISH signal amplification on AgarCyto sections according to another embodiment of the invention.

Figure 7 shows the results of CARD-ISH signal amplification on tissue sections containing CIN lesions according to a further embodiment of the invention.

### DETAILED DESCRIPTION OF EMBODIMENTS OF THE INVENTION

According to one embodiment of the invention, the amplification of ISH signals is based on the deposition of labeled tyramine, e.g., labeled tyramine ("BT"), by the activity of an enzyme, e.g., horseradish peroxidase ("HRP"), at the site of the probe. An example of a BT amplification system useful with the present invention is described generally with respect to immunoassays and immunohistochemistry in Bobrow *et al*., Catalyzed reporter deposition, a novel method of signal amplification. I. Application to Immunoassays, J. Immunol. Methods 125: 279-285, (1989), and Adams, Biotin amplification of biotin and horseradish peroxidase signals in histochemical stains, J. Histochem. Cytochem. 40: 1457-1463 (1992). *See also*, U. S. Patent 5,196,306 to Bobrow, *et al*. This method involves utilizing an analyte-dependent reporter enzyme ("ADRE") to catalyze the deposition of additional reporter on the surface in a solid-phase immunoassay. Deposition of reporter is facilitated by using a horseradish peroxidase ("HRP") ADRE to catalyze the deposition of biotin-labeled phenols. This technique will be referred to generally as catalyzed reporter deposition ("CARD").

According to the present invention, the CARD technique is applied to the amplification of in situ hybridization ("ISH") signals in order to deposit, for example, labeled tyramine ("BT") at the location of a DNA probe which has been hybridized to target DNA. This technique is referred to generally as the "CARD-ISH" signal amplification, and it results in a highly increased detection limit for both brightfield ISH and fluorescence ISH. It is understood that the CARD-ISH signal amplification technique is especially suitable for the visualization of human low copy DNA sequences and is also valuable for the detection of (oncogenic) viruses.

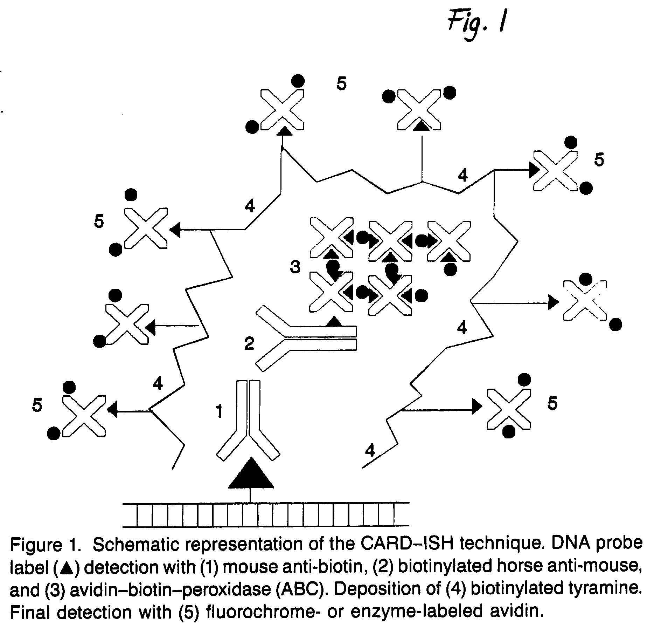

Fig. 1 is a schematic representation demonstrating the CARD-ISH technique according to an embodiment of the invention. In this case, a DNA probe label is detected with a pair of antibodies, for example, mouse antibiotin 1 and labeled horse antimouse 2, and HRP-labeled avidin-biotin-peroxidase complex ("ABC") 3. Labeled tyramine 4 is deposited at the site of the label, and final detection is provided by fluorochrome, or enzyme-labeled avidin 5.

In another embodiment of the invention, the method comprises labeling a nucleic acid, e.g., DNA, probe with a hapten. Suitable haptens include biotin, digoxygenin, and fluorescein. In one specific embodiment, the probe is labeled by nick-translation with biotin-14-dATP. Other suitable labeling techniques will occur to those with skill in the art. Next, the DNA probe label is detected with an enzyme capable of depositing tyramine, for example, an HRP detection system. There are several ways this may be accomplished. For example, in one embodiment, an avidin-biotin peroxidase complex ("ABC") is applied, and in another embodiment, an HRP-conjugated or AP-conjugated anti-hapten method is applied. More specific examples include avidin-biotin-HRP complex and avidin-biotin-AP complex. The ISH signal amplification is then achieved by deposition of labeled tyramine on the location of the probe label through enzymatic action. Suitable tyramine labels include biotin, digoxygenin, fluorescein, rhodamine, texas red, coumarin, alkaline phosphatase and beta-galactosidase. Still other suitable hapten/anti-haptens will occur to those with skill in the art. After amplification, the label source thus created is detected with fluorochrome or enzyme-labeled avidin. Of course, it will be understood that if the tyramine is labeled with fluorescein or other conjugated fluorescent reagent, then a separate detection step may be unnecessary because the fluorescence is readily observable.

According to a more specific embodiment of the present invention, a method for detecting DNA sequences is provided. This embodiment is especially useful for detecting viral sequences such as CMV, HIV, HTLV and hepatitis. In a particular embodiment, a method for detecting human papilloma virus ( 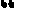 HPV 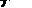 ) by in situ hybridization signal amplification based on labeled tyramine deposition is provided. In this embodiment, the method comprises the steps of labeling DNA probes specific for a type of HPV to be detected, for example by nick-translation with biotin, hybridizing the DNA probes with a target DNA; immunochemically detecting the hybridized DNA probes with an enzyme-labeled detection system, for example, mouse antibiotin, followed by labeled horse antimouse and HRP-labeled avidin-biotin complex. Next, labeled tyramine is applied by incubation to deposit additional label. Finally, the additional label is detected and the hybridized DNA is visualized, e.g., with DAB/H₂O₂. In addition to diaminobenzidin, other suitable substrates will occur to those of skill in the art, such as bromochloro indolyl phosphate-nitro blue tetrazolium.

The following examples are intended to further illustrate embodiments of the invention.

### EXAMPLE 1

### Cell and Tissue Samples

Metaphase spreads from human peripheral blood samples were prepared as described in Cremer, *et al*. Rapid Interphase and Metaphase Assessment of Specific Chromosomal Changes in Neuroectodermal Tumor Cells By In Situ Hybridization With Chemically Modified DNA Probes, Exp Cell Res 176:199, (1988). Four-µm tissue sections of a formalin-fixed, paraffin-embedded transitional cell carcinoma of the urinary bladder were mounted on aminoalkylsilane (Sigma, St. Louis, MO)-coated glass slides and heated overnight at 60°C. After dewaxing in xylene and rinsing in methanol, endogenous peroxidase activity was blocked for 20 min. at room temperature ("RT") in 1% H₂O₂-methanol. Slides were then rinsed in methanol and air-dried.

### Pre-treatment of Samples

For good accessibility of target DNA to the probes, the specimens were permeabilized as described in Kerstens, *et al*. Double-Target In Situ Hybridization in Brightfield Microscopy, J Histochem Cytochem 42:1071 (1994) which is herein incorporated by reference. Metaphase spreads were incubated for 10 min. at 37°C in 100 µg (200 U) pepsin (Sigma) per ml 0.01 M HCl. Next, the slides were rinsed in demineralized water ("MQ") and phosphate buffered saline ("PBS") and post-fixed in 1% formalin-PBS for 5 min. at RT. After rinsing in PBS and MQ, the slides were dehydrated in an ascending alcohol series and air-dried. Paraffin tissue sections were incubated in 1 M NaSCN (Merck, Darmstadt, Germany) for 10 min. at 37°C, followed by rinsing in MQ. Enzyme digestion was applied with 4 mg (8,000 U) pepsin per ml 0.2 M HCl for 10 min. at 37°C. Next, the slides were rinsed in MQ, dehydrated, and air-dried.

### DNA Probes for ISH

Centromere-associated DNA probes for chromosomes 3 (pa3.5) and 12 (pa12H8) and DNA probes HuαC (16 KB target on chromosome 14q32) and Bcl-2 (2.8 KB target on chromosome 18q21) were labeled by nick-translation with biotin-14-dATP according to the suppliers' instructions (BRL, Gaithersburg, MD).

### In Situ Hybridization

Centromere-associated DNA probe for chromosome 3 was hybridized to the paraffin tissue sections at a probe concentration of 2 ng/µl hybridization mixture containing 60% deionized formamide (Merck), 10% dextran sulfate (Sigma), 2 x SSC pH 7.0 (SSC = 0.15 M NaCl, 0.015 M Na-citrate), and 50 ng/µl herring sperm DNA (Boehringer, Mannheim, Germany). Fifteen µl of probe mixture was applied to the tissue sections under a coverslip (18 x 18mm) and sealed with rubber cement. Denaturation of probe and target DNA was performed by heating the slides in a moist chamber for 10 min. at 80°C.

ISH on metaphase spreads was performed as described by Wiegant *et al*., The Gene Encoding Human Protective Protein (PPGB) is on Chromosome 20, Genomics 10: 345 (1991). Briefly, target DNA in metaphase spreads was denatured for 3 min. at 70°C in 70% formamide (Merck) 2 x SSC, pH 7.0. Next, slides were rinsed in 70% ethanol at - 20°C, dehydrated, and air-dried. DNA probes HuαC and Bcl2 were used at a concentration of 2 ng/µl hybridization mixture. To complete repeated sequences, a 250-fold excess of Cot 1 DNA (BRL) was added to the probe mix. After denaturation of the probe DNA for 3 min. at 95°C, pre-annealing was performed for 30 min. at 37°C. Probe mix (10 µl) was applied to separately denatured metaphase spreads under a coverslip and sealed with rubber cement. The centromere-associated DNA probe for chromosome 12 was applied on metaphase spreads at a probe concentration of 0.02ng/µl hybridization mixture. Probe and target DNA were denatured simultaneously for 3 min. at 70°C.

After hybridization overnight at 37°C, coverslips were removed by immersing the slides in 2 x SSC pH 7.0 at 42°C. Post-hybridization washes (42°C) were carried out twice in 60% formamide (Merck), 2 x SSC, pH 7.0 for 5 min. and twice in 2 x SSC, pH 7.0, for 5 min. Next, the slides were rinsed in PBS-0.05% Tween 20 (PBST: Merck).

### Immunochemical Detection

For brightfield demonstration of labeled DNA probes in paraffin tissue sections, the HRP-labeled ABC method was applied, using the Elite ABC kit (Vector Laboratories: Burlingame, CA). Useful techniques for applying the ABC method are discussed in Hsu, *et al*., Use of Avidin-Biotin-Peroxidase Complex in Immunoperoxidase Techniques: Comparison Between ABC and Biotinylated Antibody (Pap) Procedures, J Histochem Cytochem 29:577 (1981). All incubation steps were performed in PBST containing 1% non-fat dry milk (PBSTM: Protifar, Nutricia, The Netherlands) for 30 min. at 37°C. Washing steps between the incubations were three times in PBST for 5 min. at RT. The tissue sections were pre-incubated with PBSTM for 15 min. at 37°C. Subsequently, mouse anti-biotin (1:100: Dakopatts, Glostrup, Denmark), labeled horse anti-mouse (1:200, HsαM-bio: Vector) and ABC (avidin 1:100, biotin-HRP 1:100 Vector) were applied.

Visualization of the probe label was accomplished by precipitating diaminobenzidine ("DAB") (Sigma) through the reaction of HRP and H₂O₂ (Merck). Therefore, the sections were incubated with 0.05% diaminobenzidine-0.15% H₂O₂ in PBS-0.65% imidazole (Merck) for 5 min. After rinsing in MQ, the DAB signal was amplified with 0.5% CuSO₄ in 0.9% NaCl for 1 min. and slides were again rinsed in MQ. Nuclei were counterstained with Mayer's hematoxylin. Finally, the samples were mounted in Permount (Fisher Scientific, Fair Lawn, N.J.).

Fluorescence detection of the DNA probe for chromosome 12 and the locus-specific DNA probes HuαC and Bcl-2 was performed according to the amplification method of Pinkel *et al*., Cytogenetic Analysis Using Quantitative, High Sensitivity, Fluorescence Hybridization, Proc. Natl. Acad. Sci., U.S.A. 83:2934 (1986). All incubations were performed in 4 x SSC-0.05% Tween 20 (4SSCT: Merck) containing 1% non-fat dry milk (4SSCTM: Protifar) for 20 min. at 37°C, and washing steps between the incubations, were three times in 4SSCT for 5 min. at RT. The metaphase spreads were pre-incubated with 4SSCTM for 10 min. at 37°C. Next, the samples were incubated with fluorescein-labeled avidin DCS (1:500: Vector). The samples were incubated for two cycles with a labeled goat anti-avidin (1:100; Vector), followed by fluorescein-labeled avidin. After rinsing in 4SSCT, 4 x SSC, and MQ, the specimens were mounted in glycerol-Tris-HCl (9:1) containing Vectashield (1:4; Vector) and 4,6-diamino-2-phenylindole (DAPI, 1 µl/ml; Sigma).

### Biotinylation of Tyramine

One hundred mg sulfosuccinimidyl-6 (biotinimide) hexanoate (NHS-LC-biotin) (Pierce: Rockford, IL) was dissolved in 40 ml 50 mM borate buffer, pH 8.0. Next, 30 mg tyramine-HCL (Sigma) was added. The solution was agitated overnight at RT and filtered. Final BT concentration was approximately 7 µM. Before application, BT was diluted in PBS.

### ISH Signal Amplification

Before amplification of the BRISH signals, the ABC technique was performed, using MαB, Hsα-M-bio, and ABC. After rinsing in PBST and PBS, BT was precipitated through the enzymatic reaction of HRP. The specimens were incubated with BT/PBS and 0.01% H₂O₂ for 5 min. at RT. The BT concentrations were 0.007 µM for the DNA probe for chromosome 3, and 0.7 µM for the locus specific DNA probes HuαC and Bcl-2. After rinsing in PBST, the biotin precipitate was detected with a second incubation of ABC (dilute ABC complex 1:4) or HRP-labeled avidin (1:50 Dakopatts) for 20 min. at 37°C. After rinsing in PBST and PBS, HRP was visualized as described above.

For amplification of the FISH signals, first the ABC (HRP) method was performed. However, mouse anti-biotin was diluted 1:10³ - 1:10⁶. BT precipitation was with BT-PBS (0.7 µM) and 0.01% H₂O₂ for 5 min. at RT. After rinsing in 4SSCT, precipitated biotin was detected with fluorescein-labeled avidin DCS (1:1,000; Vector).

### Controls

The specificity of the centromere-associated DNA probes for chromosomes 3 and 12 was defined by FISH on metaphase spreads, resulting in specific signals on the centromeric regions at chromosomes 3 and 12, respectively.

To be sure that the observed ISH signals were specific, control experiments were performed: (a) with and without biotin label in the DNA probe, and (b) with and without MαBio in the immunochemical detection. The sensitivity of the detection techniques was determined by dilution series of MαBio.

### Evaluation of ISH Signals

For evaluation of the ISH signals, a Leitz DMRB/E microscope was used with the Wild 48/52 photoequipment (Leica; Wetzlar, Germany) for photomicrography. Fluorescence filterblocks A (BP340-380/RKP400/LP430) and I3 (BP450-490/RKP510/LP515) were used for detection of DAPI and fluorescein, respectively. Photomicrographs were made with Agfa APX25 (brightfield) and Kodak EPL 400 (fluorescence).

### Results

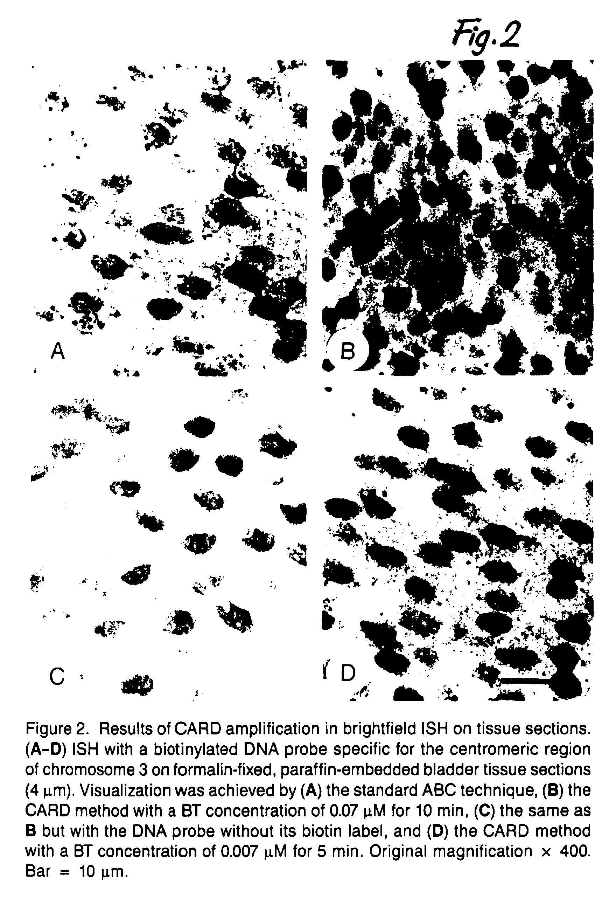

Detection of the labeled centromere-associated DNA probe specific for chromosome 3 with the standard ABC method resulted in small ISH signals. This is shown in Figure 2A. Application of the ABC technique followed by the CARD method, according to an embodiment of the invention, (BT 0.07 µM, 5 min.), resulted in strong amplification of the ISH signals. However, the amplification was so excessive that most of the nuclei were covered with DAB precipitates. This is shown in Figure 2B. The ISH signals interfered and could not be observed separately. The specificity of this reaction is shown in Figure 2C. Here, an unlabeled DNA probe was used in combination with CARD-ISH, showing no ISH signals in the nuclei. By diluting the primary antibody, mouse anti-biotin (MαBio), the ISH signals became smaller, but also more irregular in size. MαBio could be diluted 1:10⁴ before the signals disappeared. It was also possible to adjust the degree of amplification by varying the concentration and reaction time of BT. Figure 2D illustrates the results achieved according to an embodiment of the invention with 0.007 µM BT incubation for 5 min. at RT. BT concentrations higher than 0.015 µM and incubation times longer than 5 min. tend to result in excessive amplification.

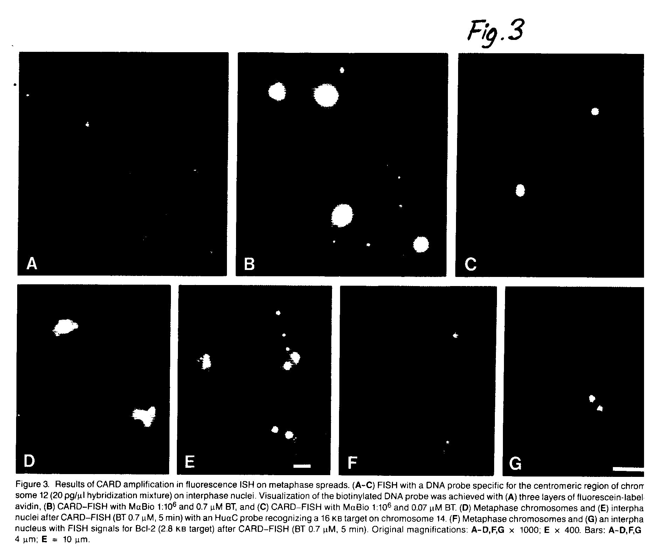

According to another embodiment, the CARD amplification is applied to FISH. In a specific embodiment, centromere-associated DNA probe specific for chromosome 12 at a concentration of 20 pg/µl hybridization mixture was hybridized to metaphase spreads. The fluorescence detection system, using three layers of fluorescein-labeled avidin, resulted in small and weak ISH signals as shown in Figure 3A. Applying more layers of avidin-fluorescein resulted in high background staining (results not shown). Detection of DNA probe label with the CARD amplification, using the BT concentration and incubation time as described above, was performed. Visualization of precipitated biotin was achieved with fluorescein-labeled avidin. However, the FISH signals were too weak for proper evaluation. In still another embodiment, enhancement of the BT concentration was increased to 0.7µM. This embodiment resulted in very strong amplification of the FISH signals as shown in Figure 3B. However, increased background staining was observed. Extending the BT incubation time (up to 20 min.) also enlarged the FISH signals, but the signals became patchy, which hampered good localization.

In still further embodiments, the amplification of the FISH signals is varied by diluting the primary antibody of the HRP detection system, i.e., MαBio. In embodiments with MαBio dilutions lower than 1:10³, this resulted in large ISH signals, but also high background staining due to a specific binding of MαBio to the specimen. In combination with 0.7 µM BT, the highest MαBio dilution used was 1:10⁶, resulting in still good detectable and evaluatable FISH signals as shown in Figure 3C. Omitting MαBio results in specific FISH signals (data not shown), because ABC can bind directly to the labeled DNA probe to amplify the signal. Unbiotinylated probe DNA in the CARD-FISH procedure resulted in no ISH signal.

In further embodiments, CARD-FISH is applied for visualization of low-copy DNA sequences. According to a specific embodiment, FISH was performed with a labeled HuαC probe that recognizes a 16 KB target on chromosome segment 14q32. Detection with three layers of fluorescein-labeled avidin resulted in barely visible FISH signals, even using a 100 x (NA 1.3) oil objective lens. Applying CARD-FISH with the high BT concentration (0.7 µM) and short incubation time (5 min.) resulted in a considerable amplification of the FISH signals, but probe localization was difficult. Improvement of localization was achieved by diluting MαBio up to 1:10⁵. Figure 3D shows these FISH signals on the q arms of chromosome 14. By diluting MαBio 1:10³-1:10⁴, clear FISH signals in interphase nuclei could be observed even with a 40 x (NA 0.70) objective lens as shown in Figure 3E. The Bcl-2 probe, which recognizes a DNA target of 2.8kb on chromosome 18q21, could not be visualized by conventional FISH detection methods. However, FISH signals were visualized by the CARD-FISH technique. MαBio could be diluted up to 1:10³, giving reasonable signals on the metaphase chromosomes, see Figure 3F, and small but clear FISH signals in the interphase nuclei, see Figure 3G.

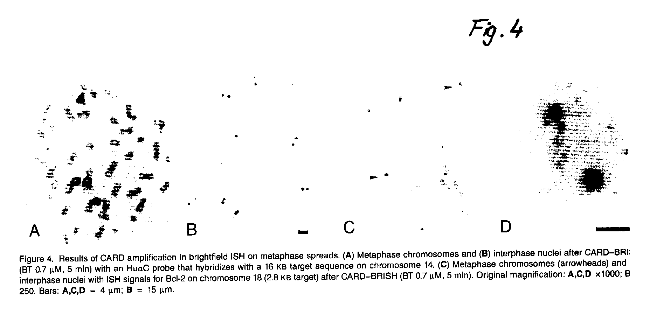

Further embodiments of the invention provide detection of low-copy DNA sequences for brightfield microscopy with CARD-BRISH. In this case, the DNA probes HuαC and Bcl-2 were used. The primary antibody MαBio 1:10³ was applied in combination with a BT concentration of 0.7 µM and incubation time of 5 min. For good localization of the ISH signals, the precipitated biotin was detected by HRP-labeled avidin instead of a second incubation with ABC. Figures 4A-4D show clear BRISH signals for HuαC and Bcl-2 on metaphase chromosomes and interphase nuclei. The 16 KB target detected by the HuαC probe could be visualized even with a x 25 (NA 0.50) objective lens (Figure 4B).

### Discussion

From the above embodiments it is understood that it is possible to control the ISH signal amplification by adjusting the BT concentration and incubation time in combination with an appropriate MαBio dilution. Generally, the BT concentration may range from about 0.0007 µM to about 7.0 µM. The incubation time may range from about 1 min. to about 10 min. Amplification of ISH signals in BRISH on paraffin tissue sections is achieved, according to versions of the present invention, by a low dilution of MαBio (1:10²) and a low concentration of BT (0.007 µM), typically providing at least a five-fold enhancement of the ISH signal size compared with standard ABC detection. In other embodiments, detection of short DNA target sequences with CARD-BRISH and CARD-FISH required higher BT concentrations. In a preferred embodiment, a MαBio dilution of 1:10² and a BT concentration of 0.007 µM is used for detection of relatively large repetitive DNA target sequences in formalin-fixed, paraffin-embedded tissue sections. For detection of short, low-copy DNA sequences, a MαBio dilution of at least 1:10³ and a BT concentration of 0.7 µM is preferred. Varying the dilution of the primary antibody (MαBio) can be used as a fine adjustment factor to obtain proper ISH signal sizes and a good signal-to-noise ratio, necessary for good evaluation. Particularly in CARD-ISH on metaphase spreads, owing to the high sensitivity of CARD-ISH, aspecific binding of MαBio resulted in background staining. To reduce this background staining and to obtain a good signal-to-noise ratio, the dilution of MαBio in CARD-ISH on metaphase spreads must be higher (at least 1:10³) than in conventional ISH techniques. The opportunity to accomplish variable ISH signal sizes and a good signal-to-noise ratio indicates that CARD-ISH is a very flexible technique for amplification of ISH signals.

### EXAMPLE 2

### Preparation of Cell and Tissue Samples

The human cervical carcinoma cell lines CaSki (ATCC; CRL 1550) containing about 500 integrated copies of HPV 16 DNA, HeLa (ATCC; CCL2) containing about 20-50 integrated copies of HPV 18 DNA, and SiHa (ATCC; HTB35) containing 1-3 integrated copies of HPV 16 DNA, are used for the determination of the sensitivity and specificity of the CARD-ISH technique. After culturing, the cells are mixed with human lymphocytes, and these cell suspensions are fixed in 70% ethanol (-20°C) and stored at -30°C. Cell suspension samples are prepared by spotting five µl on Superfrost Plus glass slides (Merck; Darmstadt, Germany) and air-dried.

Part of the cell suspensions is, after cytocentrifugation, mixed with agar, then fixed in 4% buffered formalin and embedded in paraffin (AgarCyto).

Two formalin-fixed and paraffin-embedded tissue samples, containing cervical intraepithelial neoplastic lesions (CIN 3), that show HPV type 16 positivity by PCR analysis, are selected.

Four µm thick sections of the AgarCyto's and tissue samples are mounted on Superfrost Plus glass slides and heated overnight at 60°C. After dewaxing in xylene and rinsing in methanol, endogenous peroxidase activity is blocked for 20 min. at room temperature (RT) in 1% H₂O₂/methanol. Subsequently, slides are rinsed in methanol and air-dried.

### Pre-Treatment of Samples

For a good accessibility of target DNA to the probes, the specimens are permeabilized, as described in Kerstens *et al*., Double-Target In Situ Hybridization in Brightfield Microscopy, J Histochem Cytochem 42:1071(1994). Briefly, slides, containing ethanol fixed cells, are incubated for 10 min. at 37°C in 100µg (200 U) pepsin (Sigma, St. Louis, MO.) per ml 0.01 M Hcl. Next, the slides are rinsed in demineralized water (MQ), and the nuclei are postfixed in 1% formalin in phosphate buffered saline (PBS) for 5 min. at RT. Finally, the slides are dehydrated in an ascending alcohol series and air-dried.

Formalin-fixed and paraffin-embedded tissue sections are incubated in 1 M NaSCN (Merck, Darmstadt, Germany) for 10 min. at 80°C, followed by rinsing in MQ. Enzyme digestion is applied with 4 mg (8,000 U) pepsin per ml 0.2M Hcl for 10-30 min. at 37°C. Finally, the slides are rinsed in MQ, dehydrated and air-dried.

### DNA Probes for ISH

DNA probes specific for HPV type 16 and HPV type 18 are labeled by nick-translation with biotin-14-dATP according to the supplier's instructions (BRL, Gaithersburg, MD).

### In Situ Hybridization

A hybridization mixture is prepared containing 2 ng/µl probe DNA, 60% deionized formamide (Merck), 10% dextran sulfate (Sigma), 2 x SSC, pH 7.0 (SSC = 0.15 M NaCl, 0.015 M NaCitrate), and 50 ng/µl herring sperm DNA (Boehringer, Mannheim, Germany). Fifteen µl probe mixture are applied to the samples under a coverslip (18 x 18mm) and sealed with rubber cement. Denaturation of probe and target DNA is performed simultaneously by heating the slides in a moist chamber. Ethanol fixed cell suspensions are denatured for 3 min. at 70°C and paraffin sections for 10 min. at 80°C. After hybridization overnight at 37°C coverslips are removed by immersing the slides in 2 x SSC, pH 7.0, at 42°C. Post-hybridization washes (42°C) are carried out three times in 60% formamide (Merck) in 2 x SSC, 0.05% Tween 20, pH 7.0, for 5 min. and twice in 2 x SSC, pH 7, for 5 min. Next, the slides are rinsed in PBS/0.05% Tween 20 (PBST; Merck).

### Immunochemical Detection

For the demonstration of the hybridized DNA probes the HRP labeled avidin-biotin-complex (ABC) method is applied, using the Elite ABC-kit (Vector Laboratories, Burlingame, CA). Immunochemical detection is performed using mouse anti-biotin (MαB; Dakopatts, Glostrup, Denmark), labeled horse anti-mouse (HsαM-bio; Vector) and ABC (Vector).

The probe label is visualized with 0.05% diaminobenzidin (DAB; Sigma) through the reaction of HRP with 0.15% H₂O₂ (Merck) in PBS containing 0.65% imidazole (Merck) for 5 min. The signals are intensified with 0.5% CuSO₄ in 0.9% NaCl for 1 min. Nuclei are counterstained with Mayer's hematoxylin. Finally, the samples are mounted in Permount (Fisher Scientific, Fair Lawn, NJ).

### Biotinylation of Tyramine

100 mg sulfosuccinimidyl-6-(biotinimide) hexanoate (NHS-LC-biotin; Pierce, Rockford, IL) is dissolved in 40 ml 50 mM borate buffer, pH 8.0. Next, 30 mg tyramine-HCl (Sigma) is added. The solution is agitated overnight at RT and filtered. Final labeled tyramine (BT) concentration is approximately 7 µM.

### CARD-ISH Signal Amplification

The ISH signal amplification is performed using catalyzed reporter deposition (CARD). Prior to the CARD-amplification of ISH signals, the ABC technique is performed, using MαB, HsαM-bio, and ABC. After rinsing the slides in PBST and PBS, BT is covalently bonded through the enzymatic reaction of HRP with 0.01% H₂O₂ in PBS for 2 min. at RT. For cell suspensions a BT-concentration of 0.7 µM, and for the formalin-fixed samples a BT-concentration of 0.07 µM is used. After rising in PBST, the new biotin source is detected with a second incubation of ABC for 20 min. at 37°C. Finally, the hybrid is visualized with DAB as described above.

### Negative Controls

The specificity of the ISH reactions is defined by performing CARD-ISH with an HPV type 18 DNA probe on HPV type 16 cells (CaSKi cells and SiHa cells), and ISH with the HPV type 16 probe on HeLa cells (which contain only copies of HPV type 18). None of the controls result in specific ISH signals. Furthermore, the cell line samples are mixed with human lymphocytes as internal negative controls for all HPV DNA probes.

### Evaluation of ISH Signals

For the evaluation of ISH signals a Leitz DMRB/E microscope is used with the Wild 48/52 photoequipment (Leica, Wetzlar, Germany) for microphotography. Photomicrographs are made with Agfa APX25.

### Results

According to versions of the present invention, conditions for the CARD-ISH signal amplification protocol are optimized to obtain the highest degree of signal amplification with the highest signal-to-noise ratio. In one specific embodiment, for the detection and visualization of HPV DNA sequences, the BT-concentration is 0.07 µM in PBS for 2 minutes. However, the BT concentration and incubation times may vary depending upon the specific application. Generally, depending upon the application, the BT concentration may vary from about 0.007 µM to about 7.0 µM and incubation times generally may vary from about 1 min. to about 10 minutes. Sensitivity and specificity of the ISH-reactions are determined on CaSKi (>500 copies of HPV-type 16 per cell), HeLa (20-50 copies of HPV-type 18 per cell), and SiHa (1-3 copies of HPV-type 16 per cell) cells.

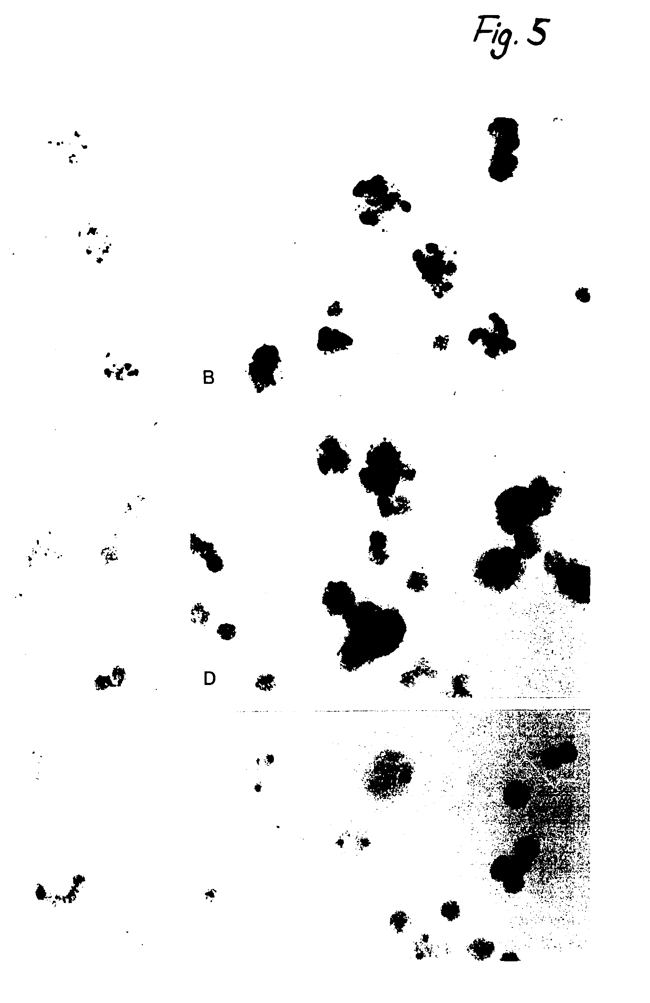

Figure 5 shows the effect of the routinely applied ISH technique in comparison to the CARD-ISH signal amplification method for the detection of HPV-DNA on cell suspension preparations of HPV containing cell lines mixed with human lymphocytes. Figure 5A demonstrates HPV-type 16 in CasKi cells, detected with the conventional ABC method. Figure 5B shows the result of the CARD-ISH signal amplification method. The human lymphocytes, present in these preparations, show no hybridization signals. Using the conventional ABC method for the detection of HPV-type 18 DNA in HeLa cells, only weak hybridization signals are observed as shown in Figure 5C. Application of the CARD-ISH signal amplification method results in very strong hybridization signals shown in Figure 5D. In Figure 5F, one to three copies of HPV-type 16 DNA in SiHa cells are visualized using the CARD-ISH method, which is difficult or impossible using the conventional ABC procedure, as shown in Figure 5E.

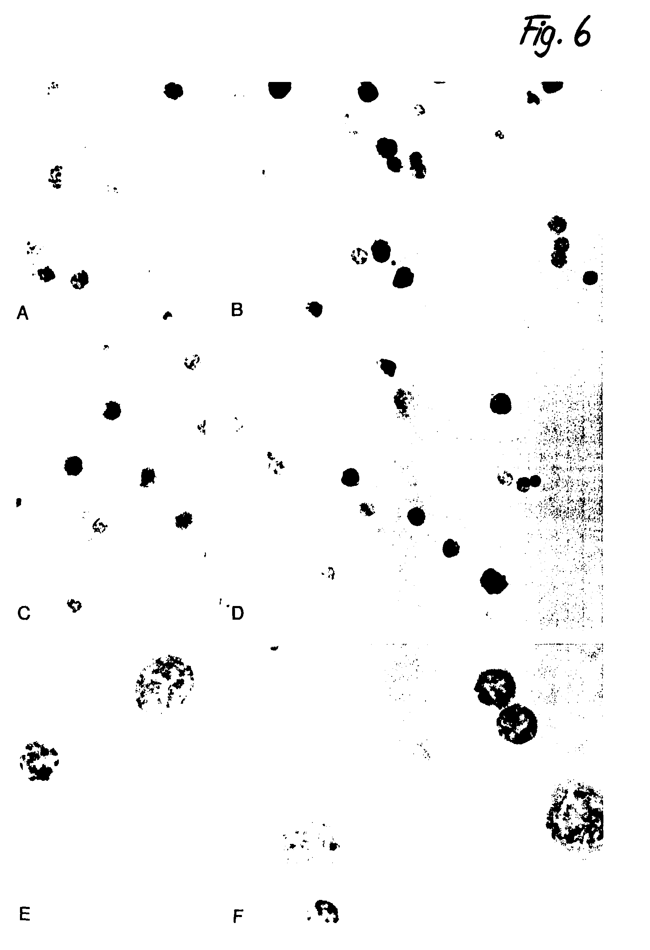

In a further embodiment, the CARD-ISH amplification method is used for the detection of HPV in paraffin embedded material having four µm thick sections of formalin fixed and paraffin embedded cell suspensions (AgarCyto's). Figure 6A-F shows the ISH results for the detection of HPV-type 16 DNA in CasKi cells (Figure 6A and B), HeLa cells (Figure 6C and D), and SiHa cells (Figure 6E and F), using the conventional ISH and CARD-ISH signal amplification method, respectively. Figures 6B, 6D, and 6F demonstrate that the hybridization signals generated after CARD-ISH are more intense compared to those generated with the conventional ISH method. Moreover, in Figure 6E no hybridization signals are visible in SiHa cells, whereas in Figure 6F one to three signals can be seen. Table 1 summarizes the ISH results of the detection of HPV DNA in cell suspensions and AgarCyto's of the three established cervical carcinoma cell-lines used.

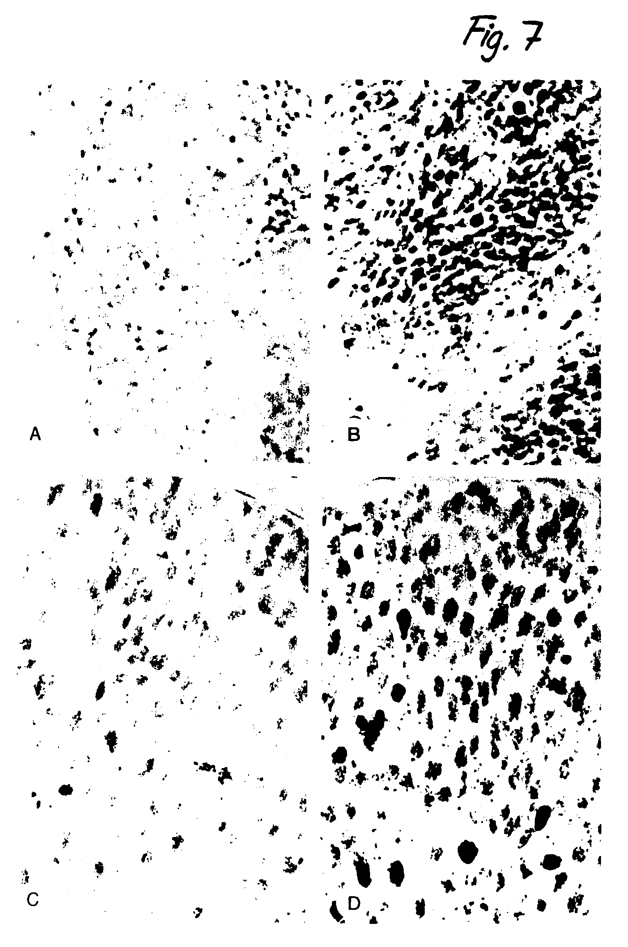

According to another version, the HPV DNA detection by the CARD-ISH signal amplification method is applied to paraffin tissue sections of two CIN 3 lesions. Figure 7 shows ISH results of these cases hybridized with HPV-type 16 DNA probe. PCR analysis of these samples demonstrates HPV-type 16 positivity (results not shown). After an immunohistochemical detection using the conventional ABC method, little HPV positivity is found. This is shown in Figures 7A and 7C. By contrast, after performance of the CARD-ISH method according to the invention, the HPV-type 16 positivity is clearly visible, see Figures 7B and 7D. Hybridization reactions of parallel tissue sections with HPV-type 18 DNA probe and biotinylated HPV-type 16 DNA probe show no ISH signals in the cells (results not shown), demonstrating the specificity of the CARD-ISH signal amplification method.

In these examples, the CARD-ISH technique is described for the detection of HPV. According to embodiments of the invention, 1 to 3 HPV copies can be detected in situ in both cell suspensions and sections of established cell lines. Furthermore, the usefulness of this new approach for routine HPV detection in formalin-fixed, paraffin-embedded cytological and histological material by CARD-ISH is demonstrated.

Also, it is to be noted that in the following examples the sensitivity and specificity of two different ISH methods using cervical cancer cell lines containing different numbers of integrated HPV genomes is compared. For this purpose, CaSKi, HeLa, and SiHa cell lines are used as a model system, because the relative amount of HPV-16 (in CaSKi: >500 copies, and in SiHa: 1-3 copies) and HPV-18 (in HeLa: 20-50 copies) per cell has been determined.

**TABLE 1**

| Comparison of Standard ISH and CARD-ISH Methods to Determine Sensitivity and Specificity of HPV-DNA Detection in Human Cervical Cancer Cell Lines | | | |
|---|---|---|---|
| Cell line (ethanol suspension) | HPV-type | ISH Result | CARD-ISH Result |
| CaSKi-cells | HPV-16 | ++ | ++++++ |
| | HPV-18 | -- | -- |
| HeLa-cells | HPV-16 | -- | -- |
| | HPV-18 | + | +++ |
| SiHa-cells | HPV-16 | -- | ++ |
| | HPV-18 | -- | -- |
| HL-controls | HPV-16 | -- | -- |
| | HPV-18 | -- | -- |
| in AgarCyto | | | |
| CaSKi-cells | HPV-16 | ++ | ++++++ |
| | HPV-18 | -- | -- |
| HeLa-cells | HPV-16 | -- | -- |
| | HPV-18 | + | +++ |
| SiHa-cells | HPV-16 | -- | ++ |
| | HPV-18 | -- | -- |
| HL = Human Lymphocytes | | | |

The above examples illustrate only specific embodiments of the invention, and are not to be considered limiting because the invention may admit to other equally effective embodiments. All patents and other documents discussed herein are hereby incorporated by reference as though set forth in full.

## Claims

1. A method for the detection of nucleic acid sequences by in situ hybridization signal amplification, the method comprising:
labeling a nucleic acid probe with a hapten;
hybridizing the nucleic acid probe with target nucleic acid sequences;
immunochemically detecting the nucleic acid probe with an enzyme-labeled detection system;
depositing labeled tyramine thereby creating additional label source;
visualizing the additional label source.

2. A method as in claim 1 wherein the nucleic acid probe is labeled with a hapten selected from the group consisting of: biotin, digoxygenin, and fluorescein.

3. A method as in claim 1 wherein the enzyme-labeled detection system comprises horseradish peroxidase ("HRP").

4. A method as in claim 1 wherein depositing labeled tyramine comprises applying tyramine labeled with a member of the group consisting of: biotin, digoxygenin, fluorescein, rhodamin, texas red, coumarin, alkaline phosphatase, and beta-galactosidase.

5. A method as in claim 4 wherein the concentration of labeled tyramine ranges from about 0.007 µM to about 7.0 µM.

6. A method as in claim 4 wherein depositing labeled tyramine comprises incubating the labeled tyramine from about 1 to about 10 minutes.

7. A method as in claim 1 wherein labeling a nucleic acid probe comprises nick-translation with biotin-14-dATP.

8. A method as in claim 1 further comprising the step of detecting the additional label source.

9. A method as in claim 8 wherein detecting the additional label source comprises applying an avidin-biotin-HRP complex or avidin-biotin-AP complex.

10. A method as in claim 8 wherein visualizing the additional label source comprises applying a substrate.

11. A method as in claim 8 wherein detecting the additional label source comprises applying HRP or AP-labeled avidin.

12. A method as in claim 8 wherein detecting the additional biotin source comprises applying a conjugated fluorescent reagent.

13. A method as in claim 10 wherein applying a substrate comprises applying diaminobenzidin.

14. A method as in claim 10 wherein applying a substrate comprises applying bromochloro indolyl phosphate-nitro blue tetrozolium.

15. A method for the detection of human papillomavirus ("HPV") by in situ hybridization signal amplification, the method comprising:
labeling DNA probes specific for a type of HPV;
hybridizing the DNA probes with target DNA;
immunochemically detecting the hybridized probes using an enzyme-labeled detection system;
applying labeled tyramine/reporter thereby creating an additional label source;
visualizing the additional label source.

16. A method as in claim 15 wherein the enzyme-labeled detection system comprises an HRP-labeled antibody.

17. A method as in claim 15 wherein the DNA probe is labeled with a hapten selected from the group of: biotin, digoxygenin, and fluorescein.

18. A method as in claim 15 wherein applying labeled tyramine comprises applying biotinylated tyramine.

19. A method as in claim 18 wherein the concentration of labeled tyramine is from about 0.007 µM to about 7.0 µM.

20. A method as in claim 18 wherein applying labeled tyramine comprises incubating the labeled tyramine from between about 1 minute to about 10 minutes.

21. A method as in claim 15 wherein labeling DNA probes comprises nick-translation with biotin-14-dATP.

22. A method as in claim 15 comprising the step of detecting the additional label source.

23. A method as in claim 22 wherein detecting the additional biotin source comprises applying a conjugated fluorescent reagent.

24. A method as in claim 22 wherein detecting the additional label source comprises applying an avidin-biotin-HRP complex or avidin-biotin-AP complex.

25. A method as in claim 22 wherein visualizing the additional label source comprises applying a substrate.

26. A method as in claim 25 wherein applying a substrate comprises applying bromochloro indolyl phosphate-nitro blue tetrazolium.

27. A method as in claim 25 wherein applying a substrate comprises applying diaminobenzidin.

28. A method for in situ hybridization signal amplification, the method comprising:
labeling a nucleic acid probe with a hapten;
hybridizing the nucleic acid probe with target nucleic acid sequences;
immunochemically detecting the nucleic acid probe with an enzyme-labeled detection system;
depositing labeled tyramine thereby creating additional label source.

29. A method as in claim 28 wherein the nucleic acid probe is labeled with a hapten selected from the group consisting of: biotin, digoxygenin, and fluorescein.

30. A method as in claim 28 wherein depositing labeled tyramine comprises depositing tyramine labeled with a member selected from the group consisting of: biotin, digoxygenin, fluorescein, rhodamin, texas red, coumarin, alkaline phosphatase, and beta-galactosidase.

31. A method as in claim 30 wherein the concentration of labeled tyramine ranges from about 0.007 µM to about 7.0 µM.

32. A method as in claim 30 wherein depositing labeled tyramine comprises incubating the labeled tyramine from about 1 to about 10 minutes.

33. A method as in claim 28 wherein labeling a nucleic acid probe comprises nick-translation with biotin-14-dATP.

34. A method as in claim 28 comprising the step of detecting the additional label source.

35. A method as in claim 34 wherein detecting the additional label source comprises applying an avidin-biotin-HRP complex or avidin-biotin-AP complex.

36. A method as in claim 34 wherein detecting the additional label source comprises applying HRP or AP-labeled avidin.

37. A method as in claim 34 wherein detecting the additional biotin source comprises applying a conjugated fluorescent reagent.

38. A method as in claim 28 wherein the enzyme-labeled detection system comprises horseradish peroxidase ("HRP").
